# NEW EUROPEAN PATENT SPECIFICATION

(11) **EP 0 823 439 B2**
(45) Date of publication and mention of the opposition decision: **16.02.2005**
(45) Mention of the grant of the patent: 24.01.2001
(21) Application number: 96305843.3
(22) Date of filing: 09.08.1996
(51) Int. Cl.: C08B 30/00, A61K 47/36, A61K 47/02, C08B 31/10, C08B 31/18

(54) **Improvements in or relating to agglomeration of starch**
Verbesserungen bei der Agglomeration von Stärke
Améliorations dans ou relatifs aux agglomérats d'amidon

(43) Date of publication of application: 11.02.1998
(73) Proprietor: Erawan Pharmaceutical Research and Laboratory Company Limited, Bangkok 10100 (TH)
(72) Inventor: Vongsurakrai, Varatus, Sumpanthawong, Bangkok 10100 (TH); Vongsurakrai, Aupakit, Sumpanthawong, Bangkok 10100 (TH); Varavinit, Saiyavit, Sumpanthawong, Bangkok 10100 (TH)
(74) Representative: Lipscombe, Martin John

(56) References cited:
- EP-A- 0 326 517
- US-A- 3 725 556
- PATENT ABSTRACTS OF JAPAN vol. 018, no. 222 (C-1193), 21 April 1994 & JP-A-06 016822 (SEKISUI PLASTICS CO LTD), 25 January 1994,

## Description

### Field of the Invention:

The invention is in the categories of Chemistry and Physics and involves the evaporation of water from starch and the binding of starch granules and silicon dioxide to form agglomerated particles. In particular the invention relates to such agglomerated particles, a method of making the particles, and compositions comprising the particles.

### Background of the Invention:

Processed starches in general do not have good "flowability" (i.e. have poor flow characteristics). This problem creates difficulties in handling and further processing of the starch. There is therefore a need for starch particles having improved flow characteristics.

US 3,725,556 discloses a method of making a pharmaceutical composition which involves the preparation of a pharmaceutically inert tablet premix. The premix is made by: foaming (with an inert gas) a suspension of 1-20% w/w silicon dioxide or aluminium oxide, 60-98% w/w of an inert filler (such as water-insoluble rice starch or corn starch) and 1-20% w/w of a water-soluble binder (such as a water-soluble starch), and then spray-drying the resulting suspension. The prior art thus teaches that use of a binder is an essential feature.

The present invention resides in the formation of starches (preferably rice starch) into spherical agglomerates by mixing the starches with silicon dioxide. The larger size and spherical shape of these agglomerates provide them with improved flowability that enables them to be used as a direct compression filler in tablet and capsule formulations. Tablets formed from these agglomerates exhibit good hardness, low friability, and favourable disintegration and dissolution characteristics. The agglomerates can also improve the flowability of powder-like substances which by themselves have poor flowability, by mixing the agglomerates with the powder so that the agglomerates serve as a carrier for the powder.

### Summary of the Invention

The present inventors have found that the flow characteristics of starch granules can be improved by agglomeration of the starch granules with silicon dioxide to form a substantially spherical agglomerated particle.

In a first aspect therefore, the invention provides an agglomerated, substantially spherical particle, consisting of up to 95 % unmodified starch and/or low-protein flour; 0.1-50% silicon dioxide; and 5-15% water.

Compositions comprising agglomerated particles in accordance with the invention are found to have excellent flow characteristics. Thus, in another aspect, the invention provides a composition comprising a plurality of agglomerated particles in accordance with the invention. The agglomerated particles are found to be particularly useful when mixed with substances (especially powder-like substances) that themselves have poor flow . characteristics, such that the particles in accordance with the invention can serve as a carrier to improve flowability. Thus the composition may take the form of a free-flowing powder.

Alternatively, because of their good flowability, the agglomerated particles of the invention may be easily handled and used, for example, as a direct compression filler in tablet or capsule formulations. Such tablets/capsules are found to have desirable qualities of hardness, low friability and desirable disintegration/dissolution characteristics. Thus the composition in accordance with the invention may be, for example, a tablet or capsule.

The agglomerated particle preferably comprises rice starch and/or low-protein rice flour. In addition, the particles of the invention may also comprise from 1 to 25% starch of a different composition. In such embodiments, the rice starch and/or low-protein rice flour content of the particle is preferably in the range 30-85%. The other starch is typically selected from one or more of the following:- tapioca starch, potato starch, corn starch, wheat starch, sago starch, and bean starch.

Generally, it is preferred that the starch and/or low-protein flour used in the particles has an amylose content of 0-40% and a protein content of 0.2-5.0% (a flour having a protein content of 0.2-5.0% being considered as "low-protein" flour), and is preferably rice starch and/or low-protein rice flour.

Particles in accordance with the invention may generally be prepared from a slurry of starch and/or low-protein flour and silicon dioxide and water. Preferably the slurry is treated in such a way as to cause loss of water from the mixture (e.g. by evaporation). Conveniently the slurry is converted into particles according to the invention by use of a spray drier, preferably using a centrifugal spray head, although other drying methods known to those skilled in the art may be employed.

### Detailed Description of the Invention:

In accordance with this invention, small granules of rice starch are mixed with silicon dioxide and water to form a slurry. Once formed, the slurry is stirred thoroughly, then dried by conventional means, a notable example being spray drying by the use of a centrifugal spray head at elevated temperature. Centrifugal spray heads are conventional equipment well known in the art of spray-drying. The drying of the slurry is generally performed at a temperature in the range 80°C-300°C. The duration of drying will depend on the precise temperature employed.

The rice starch used to form the slurry can be unmodified rice starch as well as rice starch that has been chemically modified, physically modified, or both.

Rice flour generally has a protein content of about 6.5% to 7% by weight. For best results in the formation of agglomerates in accordance with this invention, however, rice starch having a protein content in the range of 0.2% to 5.0% is preferred. A protein content in this range can be achieved by soaking the rice in a dilute sodium hydroxide solution, then milling the rice with dilute sodium hydroxide, and washing off the rice protein from the slurry, for example by centrifugal separation. The resulting low-protein rice flour can then be dried by conventional methods such as flash drying, and in preferred embodiments of the invention, the dried particles can be screened by use of a 100-mesh sieve. Rice flour treated by these methods will have a lower protein content and a whiter appearance when compared to untreated flour.

The flowability of other starches which by themselves have poor flowability can be improved by mixing the agglomerates with the powder so that the agglomerates serve as a carrier for the powder. Examples of these other starches are tapioca starch, potato starch, corn starch, maize starch, sago starch, and bean starch, as well as mixtures of two or more of these starches. Preferably, these additional starches will not exceed 25% by weight of the total composition, and most preferably will fall within the range of 1-25% by weight when present. The rice flour and/or rice starch used to form the slurry preferably has an amylose content in the range of 0% to 40%.

The relative amounts of rice starch, silicon dioxide and water can vary considerably within the scope of this invention. Preferred formulations are as follows, all percents by weight:

| | | |
|---|---|---|
| 1. | 40-95% | rice starch and/or low-protein rice flour |
| | 0.1-50% | silicon dioxide |
| | 5-15% | water |
| 2. | 30-85% | rice starch and/or low-protein rice flour |
| | 0-25 % | tapioca starch |
| | 0.1-50% | silicon dioxide |
| | 5-15% | water |

The particle sizes of the agglomerates are greater than the particles sizes of the starch granules used as a starting material, but the agglomerate size is not critical and can vary. In preferred embodiments of the invention, the agglomerate particle size is in the range of 50-200 microns in diameter, although agglomerates having a diameter of 100 microns or greater can be obtained by using a slurry of rice starch, silicon dioxide and water in which the rice starch constitutes 5-50% of the slurry (on a dry weight basis).

The formation of the agglomerates into tablets, capsules or other formulations is achieved by conventional methods well known in the pharmaceutical industry.

The invention will be further described by way of illustrative example and with reference to the accompanying drawings, in which:

Figure 1 is a photograph showing small granules of rice starch mixed with silicon dioxide and agglomerated into spherical shape. The photograph was taken using an electron microscope at 200 times magnification.

Figure 2 is a photograph showing in greater detail the central area of Figure 1. The photograph was taken using an electron microscope at 750 times magnification.

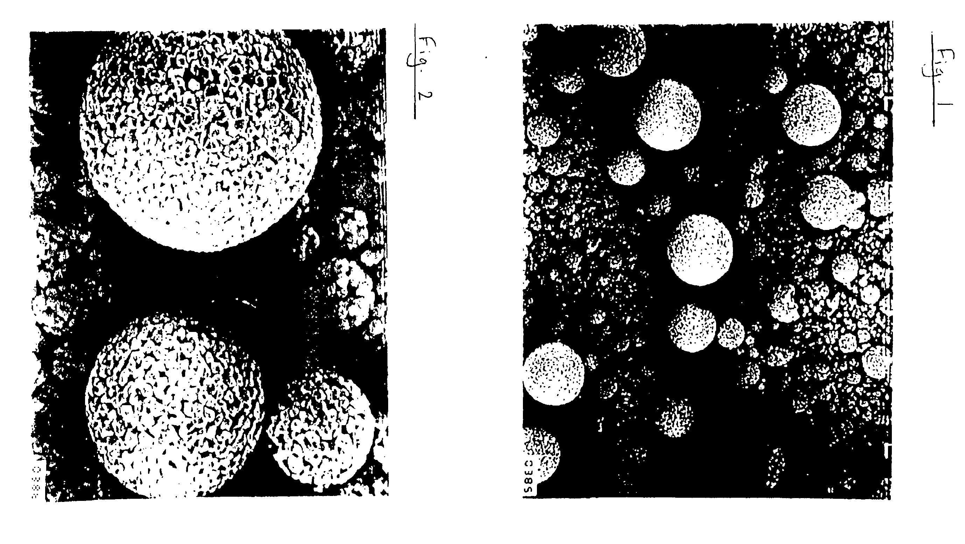

### Examples

The following examples are sample slurry compositions (containing about 50% water) for feeding into e.g. a spray drier. Drying of the slurry by the spray drier results in the formation of agglomerated particles in accordance with the invention, having a reduced water content in the range 5-15%.

### Example 1

A slurry is prepared by combining the following ingredients (all percents by weight):

| | |
|---|---|
| Rice starch or low-protein rice flour | 50% |
| Silicon dioxide | 1% |
| Water | 49% |

Agglomerates are formed from this slurry by spray drying.

### Example 2 (outside the scope of the invention)

A slurry is prepared by combining the following ingredients (all percents by weight):

| | |
|---|---|
| Rice starch or low-protein rice flour | 45% |
| Chemically and/or physically modified rice starch | 5 % |
| Silicon dioxide | 1 % |
| Water | 49% |

The chemically modified starch is starch that has been cross linked, acetylated, oxidized or modified by other types of known chemical modification. The physically modified starch is starch that has been cooked and dried by drum drying, then milled into a powder having a particle size of 60-100 mesh by appropriate sieves. Once the slurry is formed, it is converted to agglomerates by spray drying.

### Example 3 (outside the scope of the invention)

A slurry is prepared by combining the following ingredients (all percents by weight):

| | |
|---|---|
| Rice starch or low-protein rice flour | 35% |
| Chemically and/or physically modified rice starch | 5% |
| Silicon dioxide | 10% |
| Water | 50% |

The chemically and/or physically modified rice starch is prepared as described in Example 2, and the slurry is converted to agglomerates by spray drying.

### Example 4 (outside the scope of the invention)

A slurry is prepared by combining the following ingredients (all percents by weight):

| | |
|---|---|
| Rice starch or low-protein rice flour | 25% |
| Chemically and/or physically modified rice starch | 5% |
| Silicon dioxide | 20% |
| Water | 50 % |

The chemically and/or physically modified rice starch is prepared as described in Example 2, and the slurry is converted to agglomerates by spray drying.

### Example 5 (outside the scope of the invention)

A slurry is prepared by combining the following ingredients (all percents by weight):

| | |
|---|---|
| Rice starch or low-protein rice flour | 25% |
| Chemically and/or physically modified tapioca starch | 5% |
| Silicon dioxide | 20% |
| Water | 50% |

The chemically and/or physically modified tapioca starch is prepared in the same manner as described in Example 2 for the corresponding modified rice starch, and the slurry is converted to agglomerates by spray drying.

### Example 6

A slurry is prepared by combining the following ingredients (all percents by weight):

| | |
|---|---|
| Rice starch or low-protein rice flour | 44% |
| Tapioca starch | 5% |
| Silicon dioxide | 1% |
| Water | 50% |

The slurry is converted to agglomerates by spray drying.

### Example 7 (outside the scope of the invention)

A slurry is prepared by combining the following ingredients (all percents by weight):

| | |
|---|---|
| Rice starch or low protein rice flour | 39 % |
| Tapioca starch | 5 % |
| Silicon dioxide | 1 % |
| Chemically and/or physically modified tapioca starch | 5 % |
| Water | 50% |

The chemically and/or physically modified tapioca starch is prepared in the same manner as described in Example 2 for the corresponding modified rice starch, and the slurry is converted to agglomerates by spray drying.

## Claims

1. An agglomerated, substantially spherical particle, consisting of up to 95% unmodified starch and/or low-protein flour; 0.1-50% silicon dioxide; and 5-15% water.

2. A particle according to claim 1, comprising 40-95% rice starch and/or low-protein rice flour.

3. A particle according to claim 1, comprising 30-85% rice starch and/or low-protein rice flour, and additionally comprising 1-25% unmodified starch of a different composition which is derived from tapioca, potato, corn, wheat, sago or bean.

4. A particle according to any one of the preceding claims having a diameter in the range 50-200 microns.

5. A particle according to any one of the preceding claims, comprising 30-95 % rice starch and/or low-protein rice flour having a protein content of 0.2-5.0% and an amylose content of 0-40%.

6. A particle according to any one of the preceding claims, prepared by spray drying an aqueous slurry of the constituents of the particle.

7. A method of making a particle according to any one of the preceding claims, comprising forming an aqueous slurry consisting of unmodified starch and/or low-protein flour, silicon dioxide and water, and drying the slurry by spray-drying.

8. A composition comprising a plurality of particles in accordance with any one of claims 1-6.

## Patentansprüche

1. Agglomerierte, im wesentlichen sphärische Partikel, bestehend aus bis zu 95% unmodifizierter Stärke und/oder proteinarmem Mehl, 0,1 - 50% Siliciumdioxid und 5 - 15% Wasser.

2. Partikel nach Anspruch 1, umfassend 40 - 95% Reisstärke und/oder proteinarmes Reismehl.

3. Partikel nach Anspruch 1, umfassend 30 - 85% Reisstäkre und/oder proteinarmes Reismehl, und zusätzlich umfassend 1 - 25% unmodifizierte Stärke einer anderen Zusammensetzung, die von Tapioka, Kartoffeln, Getreide, Weizen, Sago oder Bohnen stammt.

4. Partikel nach einem der vorausgehenden Ansprüche, mit einem Durchmesser im Bereich von 50 - 200 Mikron.

5. Partikel nach einem der vorausgehenden Ansprüche, umfassend 30 - 95% Reisstärke und/oder proteinarmes Reismehl mit einem Proteingehalt von 0,2 - 5,0% und einem Amylose-Gehalt von 0 - 40%.

6. Partikel nach einem der vorausgehenden Ansprüche, hergestellt durch Sprühtrocknen eines wässrigen Breies der Bestandteile der Partikel.

7. Verfahren zur Herstellung von Partikeln nach einem der vorausgehenden Ansprüche, umfassend die Herstellung eines wässrigen Breies, bestehend aus unmodifizierter Stärke und/oder proteinarmem Mehl, Siliciumdioxid und Wasser, und Trocknen des Breies durch Sprühtrocknen.

8. Zusammensetzung, umfassend eine Mehrzahl von Partikeln nach einem der Ansprüche 1 - 6.

## Revendications

1. Particule agglomérée, sensiblement sphérique, étant constituée de jusqu'à 95 % d'amidon non modifié et/ou de farine à faible teneur en protéine ; de 0,1 à 50 % de dioxyde de silicium ; et de 5 à 15 % d'eau.

2. Particule selon la revendication 1, comprenant 40 à 95 % d'amidon de riz et/ou de farine de riz à faible teneur en protéine.

3. Particule selon la revendication 1, comprenant 30 à 85 % d'amidon de riz et/ou de farine de riz à faible teneur en protéine, et comprenant de plus 1 à 25 % d'amidon non modifié d'une composition différente qui est dérivée du tapioca, de la pomme de terre, du maïs, du froment, du sagou ou du haricot.

4. Particule selon l'une quelconque des revendications précédentes possédant un diamètre dans la gamme de 50 à 200 micromètres.

5. Particule selon l'une quelconque des revendications précédentes, comprenant 30 à 95 % d'amidon de riz et/ou de farine de riz à faible teneur en protéine possédant une teneur en protéine de 0,2 à 5,0 % et une teneur en amylose de 0 à 40 %.

6. Particule selon l'une quelconque des revendications précédentes, préparée en séchant par pulvérisation une pâte aqueuse des constituants de la particule.

7. Procédé de fabrication d'une particule selon l'une quelconque des revendications précédentes, comprenant la formation d'une pâte aqueuse étant constituée d'amidon non modifié et/ou de farine à faible teneur en protéine, de dioxyde de silicium et d'eau, et le séchage de la pâte à l'aide d'un séchage par pulvérisation.

8. Composition comprenant une pluralité de. particules conformément à l'une quelconque des revendications 1 à 6.
